# EUROPEAN PATENT APPLICATION

(11) **EP 4 151 108 A1**
(43) Date of publication of application: **22.03.2023**
(21) Application number: 20934918.2
(22) Date of filing: 15.05.2020
(51) Int. Cl.: A24F 47/00

(54) **ELECTRONIC ATOMIZATION DEVICE AND ATOMIZER THEREOF**

(71) Applicant: Shenzhen Smoore Technology Limited, Shenzhen, Guangdong 518102 (CN)
(72) Inventor: LEI, Guilin, Shenzhen, Guangdong 518102 (CN); JIANG, Ru, Shenzhen, Guangdong 518102 (CN)
(74) Representative: De Arpe Tejero, Manuel
(86) International application number: PCT/CN2020/090649
(87) International publication number: WO 2021/227061

(57) **Abstract**

An electronic atomization device and an atomizer (10) thereof. The atomizer (10) comprises an air inlet (11), an air outlet (12) and an airflow channel (13). The atomizer (10) comprises a first capillary liquid-absorbing structure (15) and a second capillary liquid-absorbing structure (16); once accumulated liquid absorbed by the first capillary liquid-absorbing structure (15) reaches a threshold value, the accumulated liquid in the first capillary liquid-absorbing structure (15) then enters the second capillary liquid-absorbing structure (16) and is absorbed by the second capillary liquid-absorbing structure (16). There is a gap between the first capillary liquid-absorbing structure (15) and the second capillary liquid-absorbing structure (16), air entering from the air inlet (11) passes successively through the gap and the first capillary liquid-absorbing structure (15) to arrive at an atomization core (14). The liquid leakage prevention effect of the atomizer (10) is improved.

## Description

### TECHNICAL FIELD

The present disclosure relates to the field of atomization device technology, and in particular to an electronic atomization device and an atomizer thereof.

### BACKGROUND

At present, an air inlet of an electronic atomization device such as an e-cigarette is generally provided at a bottom of an atomization cavity, and external air enters the atomization cavity from the air inlet and then reaches a mouthpiece from an air outlet channel after being mixed with an aerosol-generating material atomized in the atomization cavity. However, the atomized aerosol-generating material is easily condensed in the electronic atomization device to defin droplets, and the aerosol-generating material droplets defined through condensation are easily leaked from the air inlet of the electronic atomization device to cause liquid leakage. As a result, the existing electronic atomization device has a relatively poor liquid leakage-proof effect.

### SUMMARY OF THE INVENTION

Therefore, a first technical solution provided in the present disclosure is an atomizer, including: an air inlet; an air outlet; an airflow channel in communication with the air inlet and the air outlet, respectively, an atomization core is disposed in the airflow channel; a first capillary liquid absorbing structure and a second capillary liquid absorbing structure, the first capillary liquid absorbing structure and the second capillary liquid absorbing structure are disposed in the airflow channel provided between the air inlet and the atomization core,and the first capillary liquid absorbing structure is disposed between the atomization core and the second capillary liquid absorbing structure; and a gap is defined between the first capillary liquid absorbing structure and the second capillary liquid absorbing structure, so as to allow air entering from the air inlet to reach the atomization core after flowing through the gap and the first capillary liquid absorbing structure sequentially.

A second technical solution provided in the present disclosure is an electronic atomization device, including: a main unit and an atomizer, the main unit is connected to the atomizer, and the atomizer includes an air inlet, an air outlet; an airflow channel in communication with the air inlet and the air outlet respectively, an atomization core is disposed in the airflow channel; a first capillary liquid absorbing structure and a second capillary liquid absorbing structure, the first capillary liquid absorbing structure and the second capillary liquid absorbing structure is disposed in the airflow channel provided between the air inlet and the atomization core, the first capillary liquid absorbing structure is disposed between the atomization core and the second capillary liquid absorbing structure; and a gap is defined between the first capillary liquid absorbing structure and the second capillary liquid absorbing structure, so as to allow air entering from the air inlet to reach the atomization core after flowing through the gap and the first capillary liquid absorbing structure sequentially.

### BRIEF DESCRIPTION OF THE DRAWINGS

Accompanying drawings herein are incorporated into this specification and constitute a part of this specification, show embodiments that conform to present disclosure, and are used for describing a principle of present disclosure together with this specification. In addition, the accompanying drawings and literal descriptions are not intended to limit the scope of the idea of present disclosure in any manner, but explain the concept of present disclosure by referring to specific embodiments for a person skilled in the art
FIG. 1 is a schematic structural view of an embodiment of an atomizer according to a present disclosure;
FIG. 2 is a schematic cross-sectional structural view of the atomizer shown in FIG. 1 in a direction A-A;
FIG. 3 is a part of the schematic structure view of the atomizer shown in FIG. 2;
FIG. 4 is a schematic structural view of a second carrier of an embodiment according to the present disclosure;
FIG. 5 is a schematic structural exploded view of an atomization core, a first carrier, and a second carrier of an embodiment according to the present disclosure;
FIG. 6 is a schematic cross-sectional structural view of the atomizer shown in FIG. 1 in a direction B-B; and
FIG. 7 is a schematic structural view of an electronic atomization device of an embodiment according to the present disclosure.

### DETAILED DESCRIPTION

To make the objectives, technical solutions, and advantages of present disclosure clearer, the following clearly and completely describes the technical solutions in the embodiments of present disclosure with reference to the embodiments of present disclosure. Apparently, the described embodiments are some rather than all of the embodiments of present disclosure. All other embodiments obtained by a person of ordinary skill in the art based on the embodiments of present disclosure without creative efforts shall fall within the protection scope of present disclosure. The following embodiments and features in the embodiments may be mutually combined in a case that no conflict occurs.

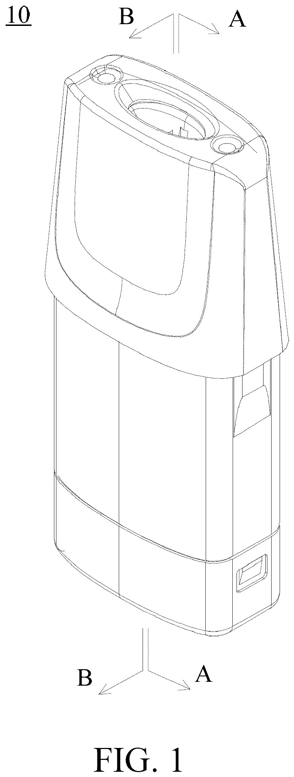

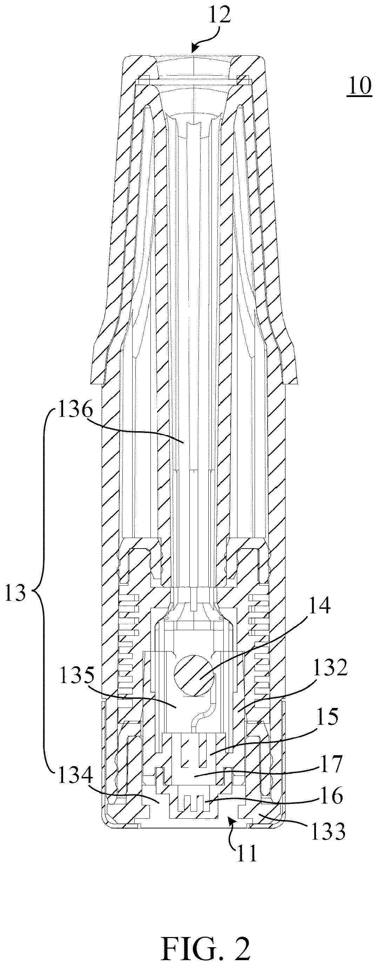

Referring to FIG. 1 and FIG. 2, FIG. 1 is a schematic structural view of an embodiment of an atomizer according to a present disclosure, and FIG. 2 is a schematic cross-sectional structural view of the atomizer shown in FIG. 1 in a direction A-A.

In an embodiment, the atomizer 10 may be in a form such as an e-cigarette. Certainly, the atomizer may also be a medical atomization device applied to the medical field. An e-cigarette as an example is provided below by using the atomizer 10 in a form of an e-cigarette as an example, which is not intended to limit the present disclosure.

In an embodiment, the atomizer 10 includes an air inlet 11, an air outlet 12, and an airflow channel 13. The airflow channel 13 is in communication with the air inlet 11 and the air outlet 12 respectively, and an atomization core 14 is disposed in the airflow channel 13, the atomization core 14 is configured to atomize an aerosol-generating material (for example, e-liquid or medical liquid) in the atomizer 10.

A position of the air inlet 11 is a position where air enters the atomizer 10. When a user sucks, external air enters the airflow channel 13 from the air inlet 11 so as to carry the aerosol-generating material atomized by the atomization core 14 in the airflow channel 13 to the air outlet 12, and output the aerosol-generating material to the user along the air outlet 12 for suction by the user.

For example, the atomization core 14 may be a porous heating body, which absorbs the aerosol-generating material through capillary force and generates heat to atomize the aerosol-generating material. Preferably, the atomization core 14 may be a porous ceramic heating body on which a heating film may be further disposed. Certainly, in some other embodiments of the present disclosure, the atomization core 14 may also be designed as a fiber cotton in cooperation with a heating wire, which is not limited herein.

The atomizer 10 of the present disclosure further includes a first capillary liquid absorbing structure 15 and a second capillary liquid absorbing structure 16. The first capillary liquid absorbing structure 15 and the second capillary liquid absorbing structure 16 are disposed in the airflow channel 13 is defined between the air inlet 11 and the atomization core 14, and the first capillary liquid absorbing structure 15 is disposed between the atomization core 14 and the second capillary liquid absorbing structure 16.

It should be noted that, since the first capillary liquid absorbing structure 15 is closer to the atomization core 14 than the second capillary liquid absorbing structure 16, then the aerosol-generating material condensed in the airflow channel 13 may be first absorbed by the first capillary liquid absorbing structure 15. In addition, because the first capillary liquid absorbing structure 15 is in communication with the second capillary liquid absorbing structure 16, after an amount of accumulated liquid absorbed by the first capillary liquid absorbing structure 15 reaches a threshold, the accumulated liquid (namely, the aerosol-generating material) in the first capillary liquid absorbing structure 15 further enters the second capillary liquid absorbing structure 16, and is absorbed by the second capillary liquid absorbing structure 16.

In an embodiment, when the accumulated liquid is less in the airflow channel 13, the first capillary liquid absorbing structure 15 absorbs the aerosol-generating material through capillary force to further keep the aerosol-generating material. When relatively the accumulated liquid is much in the airflow channel 13, after the amount of accumulated liquid absorbed by the first capillary liquid absorbing structure 15 and reaches a threshold, the aerosol-generating material in the first capillary liquid absorbing structure 15 further enters the second capillary liquid absorbing structure 16, and is absorbed by the second capillary liquid absorbing structure 16 through capillary force.

Namely, the atomizer 10 of the present disclosure increases a liquid storage (namely, accumulated liquid storage, which is the same below) amount through the first capillary liquid absorbing structure 15 and the second capillary liquid absorbing structure 16, so that a risk of accumulated liquid leakage can be reduced, and a liquid leakage-proof effect of the atomizer 10 can be further improved.

In addition, the atomizer further includes a gap 17 between the first capillary liquid absorbing structure 15 and the second capillary liquid absorbing structure 16 in the embodiment. The air entering from the air inlet 11 reaches the atomization core 14 after flowing through the gap 17 and the first capillary liquid absorbing structure 15 sequentially, the air is allowed to be uniformly mixed in the gap 17 and is uniformly distributed to the first capillary liquid absorbing structure 15, and the air further flows through the first capillary liquid absorbing structure 15 and carries the atomized aerosol-generating material to be outputted to the user. Further more, the first capillary liquid absorbing structure 15 further has an airflow rectifying function, so that the flow rates and the flow directions of airflows flowing through the first capillary liquid absorbing structure 15 are relatively consistent, the atomization core 14 can be covered by the airflows can better, and the airflows in the atomizer 10 can be optimized to carry the aerosol-generating material atomized in the atomization core 14 to the air outlet 12 better, thereby providing vapor of the carried aerosol-generating material to the user better and the use experience of the user of the atomizer 10 can be improve.

In addition, during suction by the user, the aerosol-generating material absorbed in the first capillary liquid absorbing structure 15 may return to the atomization core 14 under driving of the airflows to be atomized again, thereby the utilization of the aerosol-generating material of the atomizer 10 in the embodiment can be improved.

For example, the first capillary liquid absorbing structure 15 and the second capillary liquid absorbing structure 16 may be capillary grooves having capillary force, so that the aerosol-generating material can be absorbed through capillary force. Certainly, the first capillary liquid absorbing structure 15 and the second capillary liquid absorbing structure 16 may also be other structures having capillary force, such as a structure including capillary force in a form of a frosting surface or lines defined by roughening processing, such as grinding on a surface of the airflow channel 13, namely, the first capillary liquid absorbing structure 15 and the second capillary liquid absorbing structure 16. Detailed descriptions are provided below.

In an embodiment, the first capillary liquid absorbing structure 15 includes a plurality of capillary grooves, the extending directions of the plurality of capillary grooves are parallel to each other and the cross-sectional areas thereof are the same, and the flow rates and the flow directions of airflows flowing through the capillary grooves are the same, thereby the airflow rectifying function of the first capillary liquid absorbing structure 15 can be optimized. Furthermore, the plurality of capillary grooves may extend in a direction closing to the air outlet 12.

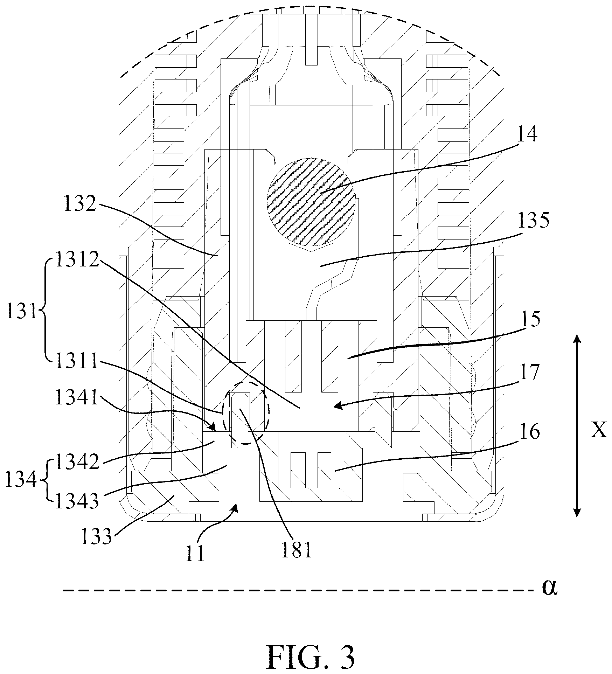

Referring to FIG. 2 and FIG. 3, FIG. 3 is a part of the schematic structure view of the atomizer shown in FIG. 2.

In an embodiment, the atomizer 10 includes a plurality of air inlets 11. FIG. 2 and FIG. 3 show a situation that the atomizer 10 includes two air inlets 11. After the air entering from the two air inlets 11 reaches the gap 17, the air can be mixed in the gap 17 and then reach the atomization core 14 through the first capillary liquid absorbing structure 15. In an embodiment, the air entering from the two air inlets 11 is uniformly mixed in the gap 17 and is uniformly distributed to the first capillary liquid absorbing structure 15, and the air cooperates with the airflow rectifying function of the first capillary liquid absorbing structure 15 to optimize the airflows in the atomizer 10 to carry the aerosol-generating material atomized at the atomization core 14 to the air outlet 12 better.

Certainly, in some other embodiments of the present disclosure, the atomizer 10 may only include one air inlet 11, and the air entering from the air inlet 11 reaches the atomization core 14 after flowing through the gap 17 and the first capillary liquid absorbing structure 15 sequentially, which is not limited herein.

Still referring to FIG. 2 and FIG. 3, in an embodiment, after the user stops sucking, vapor in the atomizer 10 may reflux. In order to prevent the refluxed vapor from directly leaking from the air inlet 11 and causing liquid leakage, a obstruction 131 is disposed in the airflow channel 13 in the embodiment, the obstruction 131 provides a barrage between the gap 17 and the air inlet 11, so as to limit the gap 17 from being in direct communication with the air inlet 11, so that the gap 17 is in communication with the air inlet 11 through the second capillary liquid absorbing structure 16.

By adoption of the foregoing method, the refluxed vapor first flows through the first capillary liquid absorbing structure 15, the condensed aerosol-generating material in the vapor is first absorbed by the first capillary liquid absorbing structure 15, and then the refluxed vapor reaching the gap 17 after flowing through the first capillary liquid absorbing structure 15 cannot directly leakage from the air inlet 11 under limitation of the obstruction 131 but enters the second capillary liquid absorbing structure 16. Through secondary absorption by the second capillary liquid absorbing structure 16, most of the aerosol-generating material in the refluxed vapor is kept in the atomizer 10 and may not leak from the atomizer 10. A risk of the refluxed vapor directly leakages from the air inlet 11 can be reduced by disposing the obstruction 131, and a risk of liquid leakage can also be reduced and the liquid leakage-proof effect of the atomizer 10 can be improved.

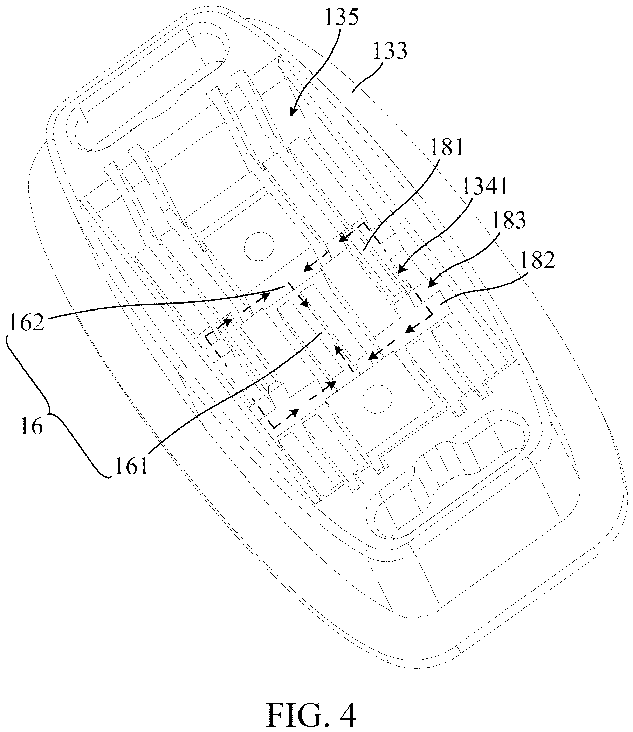

It should be noted that, the air inlet path of the atomizer 10 in the embodiment is air inlet 11-second capillary liquid absorbing structure 16-gap 17-first capillary liquid absorbing structure 15-atomization core 14-air outlet 12-user, and the air inlet path of the bottom of the airflow channel is shown by dashed arrows in FIG. 4. A vapor reflux path (after the user stops sucking) of the atomizer 10 in the embodiment is a reverse direction of the foregoing air inlet path, which is air outlet 12-atomization core 14-first capillary liquid absorbing structure 15-gap 17-second capillary liquid absorbing structure 16-air inlet 11 in an embodiment. Due to a liquid absorbing function of the second capillary liquid absorbing structure 16 and a limitation of the obstruction 131, the refluxed vapor can hardly leakage from the air inlet 11, thereby the risk of liquid leakage can be reduced greatly.

Furthermore, still referring to FIG. 2 and FIG. 3, the obstruction 131 includes a first obstruction 1311 and a second obstruction 1312, the planes in which the first obstruction 1311 and the second obstruction 1312 are located are disposed at an angle, and the gap 17 is defined by the first obstruction 1311 and the second obstruction 1312 cooperatively encircled, and after the first obstruction 1311 and the second obstruction 1312 are docked with the second capillary liquid absorbing structure 16, the gap 17 is docked with the second capillary liquid absorbing structure 16, so that the gap 17 is in communication with the air inlet 11 through the second capillary liquid absorbing structure 16. By adoption of the foregoing method, the refluxed vapor can only directly enter the second capillary liquid absorbing structure 16 through the gap 17 and cannot directly leakage from the air inlet 11 under the limitation of the first obstruction 1311 and the second obstruction 1312.

Further, still referring to FIG. 2 to FIG. 4, the airflow channel 13 includes an air inlet channel 134 and an intermediate channel 135 that are in communication with each other, the air inlet channel 134 is further in communication with the air inlet 11, and the intermediate channel 135 is further in communication with the air outlet 12. The atomization core 14, the first capillary liquid absorbing structure 15, and the second capillary liquid absorbing structure 16 are disposed in the intermediate channel 135.

Further, the airflow channel 13 may further include an air outlet channel 136, and the intermediate channel 135 is in communication with the air outlet 12 through the air outlet channel 136.

The second capillary liquid absorbing structure 16 is disposed close to an end opening 1341 of the air inlet channel 134 which is in communication with the intermediate channel 135, and another end opening of the air inlet channel 134 opposite to the end opening 1341 is the air inlet 11. A first dam 181 and a second dam 182 that are disposed around a periphery of the end opening 1341 of the air inlet channel 134 in communication with the intermediate channel 135 are disposed in the intermediate channel 135. The first dam 181 is disposed close to the gap 17 relative to the second dam 182 and the first dam 181 provides a barrage between the gap 17 and the air inlet channel 134, so as to limit the gap 17 from being in direct communication with the air inlet channel 134, so that the gap 17 is in communication with the air inlet channel 134 through the second capillary liquid absorbing structure 16. The first dam 181 may be a part of the first obstruction 1311 or the second obstruction 1312, FIG. 3 shows a situation that the first dam 181 is a part of the first obstruction 1311, and detailed descriptions are provided below.

Further, the height of the first dam 181 is higher than the height of the second dam 182 to defin an air vent 183 between the first dam 181 and the second dam 182. The air inlet channel 134 is in communication with the second capillary liquid absorbing structure 16 through the air vent 183 and is further in communication with the gap 17. As shown in FIG. 3 and FIG. 4, air entering from the air inlet channel 134 needs to flow through the air vent 183 to enter the second capillary liquid absorbing structure 16 and then reach the gap 17. A path that the air enters from the end opening 1341 of the air inlet channel and flows through the air vent 183 to enter the second capillary liquid absorbing structure 16 is shown by dashed arrows in FIG. 4, where the end opening 1341 is in communication with the intermediate channel.

FIG. 4 shows a situation that the first dam 181, the second dam 182, and the side wall of the bottom of the intermediate channel 135 cooperatively surround the end opening 1341 of the air inlet channel, where the end opening 1341 is in communication with the intermediate channel, and the second dam 182 is disposed on two sides of the first dam 181 respectively, namely, the air vent 183 is provided on two sides of the first dam 181 respectively. Namely, the air entering from the air inlet channel flows through the air vents 183 on two sides of the first dam 181 to enter the second capillary liquid absorbing structure 16.

FIG. 3 and FIG. 4 further show a situation that two opposite air inlet channels 134 are provided on the bottom of the intermediate channel 135. The gap 17 is disposed opposite to the second capillary liquid absorbing structure 16 between the two air inlet channels 134, the air entering from the two air inlet channels 134 turns to enter the second capillary liquid absorbing structure 16 between the two air inlet channels 134, and together enters the gap 17 after confluence, and the air then reaches the atomization core 14 through the first capillary liquid absorbing structure 15, so as to carry the atomized aerosol-generating material to be outputted to the user.

In addition, still referring to FIG. 4, the first dam 181 and the second dam 182 are further configured to isolate the air inlet channel from the second capillary liquid absorbing structure 16. In an embodiment, the end opening 1341 of the air inlet channel is isolated from the second capillary liquid absorbing structure 16, and the end opening 1341 is in communication with the intermediate channel. Therefore, even if an amount of the aerosol-generating material absorbed by the second capillary liquid absorbing structure 16 is relatively large, the aerosol-generating material absorbed by the second capillary liquid absorbing structure 16 may not leak from the air inlet channel, thereby the risk of liquid leakage can be reduced further.

Further, the heights of the first dam 181 and the second dam 182 are higher than the height of the second capillary liquid absorbing structure 16, so that the risk of liquid leakage can be further reduced on the basis of disposing the first dam 181 and the second dam 182.

Still referring to FIG 3, in an embodiment, an orthographic projection of the end opening 1341 of the air inlet channel 134 in communication with the intermediate channel 135 on a reference plane is located outside of an orthographic projection of the gap 17 on the reference plane, and the reference plane (as shown by a plane α in FIG. 3) is perpendicular to the direction along which the first capillary liquid absorbing structure 15 and the second capillary liquid absorbing structure 16 are opposite to each other (the direction along which the first capillary liquid absorbing structure 15 and the second capillary liquid absorbing structure 16 are opposite to each other is shown by an arrow X in FIG. 3). Namely, the reference plane is perpendicular to a central axis of the atomizer in a direct-liquid atomizer.

Namely, the air inlet channel 134 and the gap 17 in the embodiment are provided in a misaligned manner on the reference plane, so as to prevent vapor refluxed from the gap 17 from directly leaking from the air inlet channel 134 and causing liquid leakage, and the risk of liquid leakage can also be reduced and the liquid leakage-proof effect of the atomizer 10 can be improved.

The orthographic projections of the two air inlet channels 134 shown in FIG. 3 on the reference plane are located on two opposite sides of the orthographic projection of the gap 17 on the reference plane.

Still referring to FIG 3, in an embodiment, the air inlet channel 134 includes at least two first sub-channels 1342, adjacent of the first sub-channels 1342 are connected through a second sub-channel 1343, and the extending direction of the first sub-channel 1342 is different from the extending direction of the second sub-channel 1343. Namely, the air inlet channel 134 extends zigzag, and the zigzag extended air inlet channel 134 increases the difficulty that the refluxed vapor leakage from the air inlet channel 134, thereby the risk of liquid leakage can also be reduced and the liquid leakage-proof effect of the atomizer can be improved.

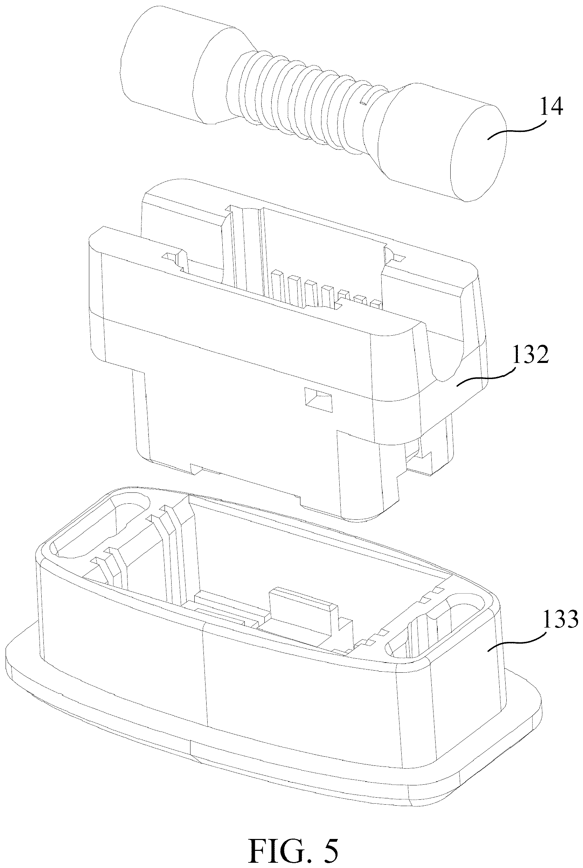

Referring to FIG. 2, FIG. 3, and FIG. 5, FIG. 5 is a schematic structural exploded view of an atomization core, a first carrier, and a second carrier of an embodiment according to the present disclosure.

In an embodiment, the atomizer 10 further includes a first carrier 132 and a second carrier 133. The first carrier 132 is docked with the second carrier 133 to define the airflow channel 13, and a cavity defined by docking between the first carrier 132 and the second carrier 133 is provided for flowing of air. The atomization core 14 and the first capillary liquid absorbing structure 15 are disposed on the first carrier 132, and the first carrier 132 is in communication with the air outlet 12. The air inlet 11 and the second capillary liquid absorbing structure 16 are disposed on the second carrier 133.

Namely, the airflow channel 13 of the atomizer 10 in the embodiment is a separated structure, which is beneficial to injection molding of various components. In an embodiment, the aerosol-generating material leaked from the atomization core 14 on the first carrier 132 is first absorbed by the first capillary liquid absorbing structure 15 on the first carrier 132. After an amount of accumulated liquid absorbed by the first capillary liquid absorbing structure 15 reaches a threshold, the aerosol-generating material in the first capillary liquid absorbing structure 15 further infiltrates into the second capillary liquid absorbing structure 16. Because the second capillary liquid absorbing structure 16 on the second carrier 133 includes a large liquid storage space, most infiltrated aerosol-generating material can be absorbed, so as to prevent the aerosol-generating material from leaking to the outside of the atomizer 10.

In an embodiment, in the foregoing embodiment, the first dam 181 is disposed on the second carrier 133, a part of the first carrier 132 which docked with the second carrier 133 and the first dam 181 form the first obstruction 1311 together in the foregoing embodiment, and as shown in FIG. 3, the second obstruction 1312 in the foregoing embodiment is also disposed on the first carrier 132.

Certainly, in some other embodiments of the present disclosure, the first carrier 132 and the second carrier 133 may also be integrally formed through 3D printing, which is not limited herein.

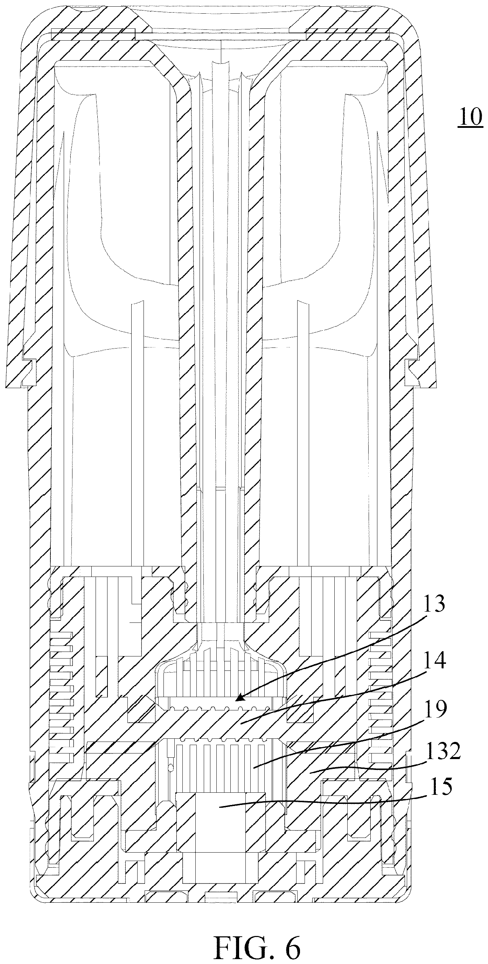

Referring to FIG. 6, FIG. 6 is a schematic cross-sectional structural view of the atomizer shown in FIG. 1 in a direction B-B.

In an embodiment, the atomizer 10 further includes a third capillary liquid absorbing structure 19. The third capillary liquid absorbing structure 19 is disposed on the part of the inner wall of the airflow channel 13 close to the atomization core 14, and the third capillary liquid absorbing structure 19 is configured to cooperate with the first capillary liquid absorbing structure 15 to absorb accumulated liquid, so that a liquid storage amount in the atomizer 10 is further increased, and the risk of liquid leakage is further reduced, which makes the liquid leakage-proof effect of the atomizer 10 is improved.

Further, the third capillary liquid absorbing structure 19 is disposed on the first carrier 132 in the foregoing embodiment. In an embodiment, the third capillary liquid absorbing structure 19 is disposed on the side wall of the inner cavity of the first carrier 132, and the first capillary liquid absorbing structure 15 is disposed at the bottom of the inner cavity of the first carrier 132. The aerosol-generating material absorbed by the third capillary liquid absorbing structure 19 and further flow toward the first capillary liquid absorbing structure 15, andis absorbed by the first capillary liquid absorbing structure 15.

It should be noted that, the first capillary liquid absorbing structure, the second capillary liquid absorbing structure, and the third capillary liquid absorbing structure may all be capillary grooves. As shown in FIG. 3, in an embodiment, the first capillary liquid absorbing structure 15 extends along its direction opposite to the second capillary liquid absorbing structure 16. Namely, the first capillary liquid absorbing structure 15 extends in a longitudinal direction. Certainly, the third capillary liquid absorbing structure may also extend in a longitudinal direction, which is not limited herein.

Still referring to FIG 4, the second capillary liquid absorbing structure 16 includes a first capillary groove 161 and a second capillary groove 162. The first capillary groove 161 and the second capillary groove 162 are in communication with each other and extend along different directions. By adoption of the foregoing method, a speed at which the second capillary liquid absorbing structure 16 absorbs the aerosol-generating material is increased, and an effect that the second capillary liquid absorbing structure 16 absorbs the aerosol-generating material is improved, thereby the risk of liquid leakage can be reduced further, and the liquid leakage-proof effect of the atomizer is improved.

In the embodiment, the plane defined by the extending directions of the first capillary groove 161 and the second capillary groove 162 is perpendicular to the central axis of the atomizer in a direct-liquid atomizer, and the capillary groove of the first capillary liquid absorbing structure extends in the direction of the central axis of the atomizer.

For example, widths of capillary grooves of the first capillary liquid absorbing structure, the second capillary liquid absorbing structure, and the third capillary liquid absorbing structure are preferably less than 1 mm, so that the first capillary liquid absorbing structure, the second capillary liquid absorbing structure, and the third capillary liquid absorbing structure include a sufficient capillary liquid absorbing capability. If the width of the capillary groove is excessively large, the capillary liquid absorbing capability of the capillary groove is relatively weak and is not sufficient to meet a use requirement. In addition, a design value of the width of the capillary groove also depends on the viscosity of the aerosol-generating material and the structure design limitation of the atomizer. In addition, a greater depth of the capillary groove indicates a greater liquid storage amount. Therefore, if the structure allows, increasing the depth of the capillary groove can make the liquid storage amount of the capillary groove being increased, thereby the risk of liquid leakage can be reduced.

In summary, according to the atomizer provided in the present disclosure, after an amount of accumulated liquid absorbed by the first capillary liquid absorbing structure reaches a threshold, the accumulated liquid in the first capillary liquid absorbing structure further enters the second capillary liquid absorbing structure and can be absorbed by the second capillary liquid absorbing structure. Namely, the atomizer of the present disclosure increases a liquid storage (namely, accumulated liquid storage) amount through the first capillary liquid absorbing structure and the second capillary liquid absorbing structure, so that the risk of accumulated liquid leakage can be reduced, and the liquid leakage-proof effect of the atomizer can be further improved.

In addition, the atomizer further includes a gap between the first capillary liquid absorbing structure and the second capillary liquid absorbing structure in the present disclosure. The air entering from the air inlet reaches the atomization core after flowing through the gap and the first capillary liquid absorbing structure sequentially, the air is allowed to be uniformly mixed in the gap and is uniformly distributed to the first capillary liquid absorbing structure, and the air further flows through the first capillary liquid absorbing structure and carries the atomized aerosol-generating material to be outputted to the user. In addition, the first capillary liquid absorbing structure further has an airflow rectifying function, so that the flow rates and the flow directions of airflows flowing through the first capillary liquid absorbing structure are relatively consistent, the airflows can better cover the atomization core, and the airflows in the atomizer can be optimized to carry the aerosol-generating material atomized at the atomization core to the air outlet better, thereby better providing vapor of the carried aerosol-generating material to the user and the user experience is improved.

Furthermore, a obstruction is disposed in the airflow channel of the present disclosure, and the obstruction provides a barrage between the gap and the air inlet, so as to prevent the refluxed vapor from leaking from the air inlet and causing liquid leakage directly.

In addition, the air inlet channel and the gap of the present disclosure are provided in a misaligned manner. Namely, an air inlet part and a main atomization airway of the atomizer of the present disclosure are provided in a misaligned manner, and prevent the vapor refluxed from the gap from leaking from the air inlet channel and causing liquid leakage directly.

In addition, during suction by the user, the aerosol-generating material absorbed in the first capillary liquid absorbing structure may return to the atomization core under driving of the airflows to be atomized again, thereby the utilization of the aerosol-generating material of the atomizer of the present disclosure can be improved.

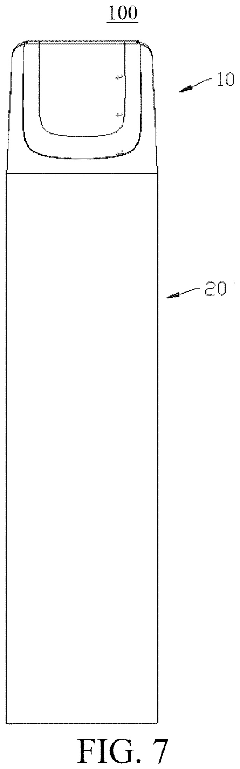

Referring to FIG. 7, FIG. 7 is a schematic structural view of an electronic atomization device of an embodiment according to the present disclosure.

In an embodiment, the electronic atomization device 100 includes an atomizer 10 and a main unit 20. The atomizer 10 is configured to heat and atomize an aerosol-generating material (for example, e-liquid). The main unit 20 is provided with a power supply and a control circuit. The atomizer 10 may be fixedly connected to the main unit 20, or may be detachably connected to the main unit 20.

The atomizer 10 includes an air inlet, an air outlet, and an airflow channel. The airflow channel is in communication with the air inlet and the air outlet respectively, and an atomization core is disposed in the airflow channel. The atomizer further includes: a first capillary liquid absorbing structure and a second capillary liquid absorbing structure, the first capillary liquid absorbing structure and the second capillary liquid absorbing structure are disposed in the airflow channel provided between the air inlet and the atomization core, and the first capillary liquid absorbing structure is disposed between the atomization core and the second capillary liquid absorbing structure; a gap is defined between the first capillary liquid absorbing structure and the second capillary liquid absorbing structure, so as to allow air entering from the air inlet to reach the atomization core after flowing through the gap and the first capillary liquid absorbing structure sequentially. The atomizer 10 has been described in detail in the foregoing embodiments, and details are not repeated herein again.

In addition, in the present disclosure, unless otherwise explicitly specified or defined, the terms such as "connect", "connection", and "stack" should be understood in a broad sense. For example, the connection may be a fixed connection, a detachable connection, or an integral connection; or a direct connection, an indirect connection through an intermediate, or internal communication between two elements or an interaction relationship between two elements. A person of ordinary skill in the art may understand the specific meanings of the foregoing terms in the present disclosure according to specific situations.

Finally, it should be noted that the foregoing embodiments are merely used for describing the technical solutions of the present disclosure, but are not intended to limit the present disclosure. Although the present disclosure is described in detail with reference to the foregoing embodiments, a person of ordinary skill in the art should understand that, modifications may still be made to the technical solutions in the foregoing embodiments, or equivalent replacements may be made to some or all of the technical features; and these modifications or replacements will not cause the essence of corresponding technical solutions to depart from the scope of the technical solutions in the embodiments of the present disclosure.

## Claims

1. An atomizer, comprising:
an air inlet;
an air outlet;
an airflow channel in communication with the air inlet and the air outlet, respectively, an atomization core is disposed in the airflow channel;
a first capillary liquid absorbing structure and a second capillary liquid absorbing structure, the first capillary liquid absorbing structure and the second capillary liquid absorbing structure are disposed in the airflow channel provided between the air inlet and the atomization core,and the first capillary liquid absorbing structure is disposed between the atomization core and the second capillary liquid absorbing structure; and
a gap is defined between the first capillary liquid absorbing structure and the second capillary liquid absorbing structure, so as to allow air entering from the air inlet to reach the atomization core after flowing through the gap and the first capillary liquid absorbing structure sequentially.

2. The atomizer of claim 1, wherein a plurality of air inlets are provided, and air entering from the plurality of air inlets flows through the first capillary liquid absorbing structure after being mixed in the gap.

3. The atomizer of claim 1, wherein the first capillary liquid absorbing structure comprises a plurality of capillary grooves, wherein extending directions of the plurality of capillary grooves being parallel to each other, and cross-sectional areas thereof being the same, and
wherein flow rates and flow directions of airflow flowing through each of the plurality of capillary grooves are the same.

4. The atomizer of claim 1, wherein an obstruction is disposed in the airflow channel, the obstruction defins blocking between the gap and the air inlet so as to limit the gap from being in direct communication with the air inlet, and
wherein the gap is in communication with the air inlet through the second capillary liquid absorbing structure.

5. The atomizer of claim 4, wherein the obstruction comprises a first obstruction and a second obstruction, wherein a planes in which the first obstruction and the second obstruction are located being disposed at an angle, and the first obstruction and the second obstruction cooperatively encircle to define the gap,
wherein, after the first obstruction and the second obstruction are docked with the second capillary liquid absorbing structure, the gap is docked with the second capillary liquid absorbing structure, and
wherein the gap is in communication with the air inlet through the second capillary liquid absorbing structure.

6. The atomizer of claim 1, wherein the airflow channel comprises an air inlet channel and an intermediate channel that are in communication with each other, the air inlet channel is in communication with the air inlet, the intermediate channel is in communication with the air outlet, and
wherein the atomization core, the first capillary liquid absorbing structure, and the second capillary liquid absorbing structure are disposed in the intermediate channel.

7. The atomizer of claim 6, wherein the second capillary liquid absorbing structure is disposed on an end opening of the air inlet channel, a first dam and a second dam are disposed around a periphery of the end opening of the air inlet channel, the end opening of the air inlet channel in communication with the intermediate channel;
wherein the first dam is disposed close to the gap relative to the second dam, and the first dam provides an barrage between the gap and the air inlet channel so as to limit the gap from being in direct communication with the air inlet channel,
wherein the gap is in communication with the air inlet channel through the second capillary liquid absorbing structure, and
wherein the first dam and the second dam are further configured to isolate the air inlet channel from the second capillary liquid absorbing structure.

8. The atomizer of claim 7, wherein a height of the first dam is higher than a height of the second dam;
wherein an air vent is defined between the first dam and the second dam, and
wherein the air inlet channel is in communication with the second capillary liquid absorbing structure through the air vent and the air inlet channel is in communication with the gap.

9. The atomizer of claim 7, wherein heights of the first dam and the second dam are higher than a height of the second capillary liquid absorbing structure.

10. The atomizer of claim 7, wherein the first dam, the second dam, and a side wall of the bottom of the intermediate channel cooperatively surround the end opening of the air inlet channel, and
wherein the end opening of the air inlet channel is in communication with the intermediate channel.

11. The atomizer of claim 6, wherein an orthographic projection of the end opening of the air inlet channel on a reference plane is located outside an orthographic projection of the gap on the reference plane;
wherein the end opening is in communication with the intermediate channel, and
wherein the reference plane is perpendicular to a direction which is opposite to a direction of the first capillary liquid absorbing structure and the second capillary liquid absorbing structure.

12. The atomizer of claim 6, wherein the air inlet channel comprises at least two first sub-channels;
wherein the adjacent of the first sub-channels are connected through a second sub-channel, and
wherein an extending direction of the first sub-channel is different from an extending direction of the second sub-channel.

13. The atomizer of claim 6, wherein two opposite air inlet channels are provided on a bottom of the intermediate channel, and
wherein the gap is provided opposite to the second capillary liquid absorbing structure between the two air inlet channels.

14. The atomizer of claim 6, wherein the airflow channel further comprises an air outlet channel, and
wherein the intermediate channel is in communication with the air outlet through the air outlet channel.

15. The atomizer of claim 1, wherein the first capillary liquid absorbing structure and the second capillary liquid absorbing structure comprise capillary grooves.

16. The atomizer of claim 15, wherein the first capillary liquid absorbing structure extends along its direction opposite to the second capillary liquid absorbing structure;
wherein the second capillary liquid absorbing structure comprises a first capillary groove and a second capillary groove, the first capillary groove and the second capillary groove being in communication with each other and extend along different directions, and
wherein a plane defined by extending directions of the first capillary groove and the second capillary groove is perpendicular to an extending direction of the first capillary liquid absorbing structure.

17. The atomizer of claim 1, further comprising:
a first carrier and a second carrier, the first carrier being docked with the second carrier to defin the airflow channel;
wherein the atomization core and the first capillary liquid absorbing structure are disposed on the first carrier, and
wherein the second capillary liquid absorbing structure is disposed on the second carrier.

18. The atomizer of claim 1, further comprising:
a third capillary liquid absorbing structure;
wherein the third capillary liquid absorbing structure is disposed on a part of the inner wall of the airflow channel that is close to the atomization core.

19. The atomizer of claim 18, wherein widths of capillary grooves of the first capillary liquid absorbing structure, the second capillary liquid absorbing structure, and the third capillary liquid absorbing structure are less than 1 mm.

20. An electronic atomization device, comprising:
a main unit; and
an atomizer, the main unit is electrically connected to the atomizer, and the atomizer comprising:
an air inlet;
an air outlet;
an airflow channel in communication with the air inlet and the air outlet respectively, an atomization core being disposed in the airflow channel;
a first capillary liquid absorbing structure and a second capillary liquid absorbing structure, wherein the first capillary liquid absorbing structure and the second capillary liquid absorbing structure being disposed in the airflow channel provided between the air inlet and the atomization core, and the first capillary liquid absorbing structure being disposed between the atomization core and the second capillary liquid absorbing structure; and
a gap is defined between the first capillary liquid absorbing structure and the second capillary liquid absorbing structure, so as to allow air entering from the air inlet to reach the atomization core after flowing through the gap and the first capillary liquid absorbing structure sequentially.
